(19) Europäisches Patentamt
European Patent Office
Office européen des brevets

(11) **EP 4 020 381 B1**

(12) **EUROPEAN PATENT SPECIFICATION**

(45) Date of publication and mention
of the grant of the patent:
**06.05.2026 Bulletin 2026/19**

(21) Application number: **20853749.8**

(22) Date of filing: **20.08.2020**

(51) International Patent Classification (IPC):
***G06T 7/00*** *(2017.01)*      ***A61B 6/00*** *(2024.01)*
***A61B 6/12*** *(2006.01)*      ***G06T 1/00*** *(2006.01)*

(52) Cooperative Patent Classification (CPC):
**A61B 6/486; A61B 6/12; A61B 6/481;
A61B 6/5235; A61B 6/5264; G06T 5/50;**
A61B 6/461; A61B 6/487; A61B 6/501; A61B 6/505;
A61B 6/54; G06T 2207/10016; G06T 2207/10116;
G06T 2207/10121; G06T 2207/20221;      (Cont.)

(86) International application number:
**PCT/JP2020/031427**

(87) International publication number:
**WO 2021/033741 (25.02.2021 Gazette 2021/08)**

(54) **X-RAY DIAGNOSIS APPARATUS, AND MEDICAL IMAGE PROCESSING METHOD**

RÖNTGENDIAGNOSEVORRICHTUNG UND VERFAHREN ZUR VERARBEITUNG
MEDIZINISCHER BILDER

APPAREIL DE DIAGNOSTIC RADIOGRAPHIQUE ET PROCÉDÉ DE TRAITEMENT D'IMAGES
MÉDICALES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.08.2019  JP 2019152360
19.08.2020  JP 2020138714**

(43) Date of publication of application:
**29.06.2022 Bulletin 2022/26**

(73) Proprietor: **Canon Medical Systems Corporation
Tochigi 324-0036 (JP)**

(72) Inventor: **ABE, Shingo
Otawara-shi, Tochigi 324-0036 (JP)**

(74) Representative: **Marks & Clerk LLP
15 Fetter Lane
London EC4A 1BW (GB)**

(56) References cited:
WO-A1-2014/192504      JP-A- 2002 333 974
JP-A- 2013 111 227      JP-A- 2018 083 010
US-A1- 2015 139 394      US-A1- 2018 082 420

**(Cont. next page)**

(52) Cooperative Patent Classification (CPC): (Cont.)
G06T 2207/20224; G06T 2207/30021

**Description**

**FIELD**

Embodiments described herein relate generally to a medical image processing apparatus, an x-ray diagnostic apparatus, and a medical image processing method.

**BACKGROUND**

X-ray diagnostic apparatuses such as an X-ray fluoroscopic apparatus and an X-ray angiography apparatus have been provided recently in which the inside of an object can be observed in real time by irradiating the object with X-rays and sequentially displaying the acquired images like moving images in real time. Further, the time-series X-ray images acquired by the X-ray diagnostic apparatus can be observed as moving images a plurality of time-series X-ray images acquired by the X-ray diagnostic apparatus can be observed as a moving image in the X-ray diagnostic apparatus or other modality after the scan in the post process.

Such X-ray diagnostic apparatus may be used for angiography using a catheter of IVR (Interventional Radiology). For example, when performing a catheter treatment, a user may display an X-ray fluoroscopic image based on X-ray imaging by an X-ray diagnostic apparatus in real time, and the procedure may be performed while checking positions of a catheter and a treatment device such as a balloon (including the indwelling position) depicted in the X-ray image. Further, after the procedure, the X-ray image may be used to confirm whether the treatment device such as the balloon has been placed at a desired position.

Generally, image processing such as background compression, signal enhancement, and gradation conversion is performed on the X-ray image in order to improve the visibility of instruments such as contrast agents and catheters depicted on X-ray images. By performing image processing such as gradation conversion, it is possible to emphasize in the X-ray image the shade of a device such as a contrast agent or a catheter, which is the target to be observed for the user.

However, when there are non-target components such as bones and diaphragms depicted in the same X-ray image, the shades of these non-target components are also emphasized by the image processing such as gradation conversion. In this case, as a result of performing image processing such as gradation conversion, the visibility of the target may be worsened by the non-target components.

US 2015/139394 A1 discloses an X-ray imaging apparatus including an X-ray source configured to radiate X-rays onto an object region, an X-ray detector configured to detect the radiated X-rays and obtain image frames of the object region based on the detected X-rays, and a filter configured to filter X-rays radiated from the X-ray source such that the X-rays incident on a region of interest (ROI) of the object region have a lower dose than a dose of X-rays incident on a non-ROI of the object region.

US 2018/082420 A1 discloses methods and systems for generating regional digital subtraction angiography (DSA) images and roadmap images with landmarks. In one embodiment, a method comprises generating a mask from a set of mask images of an anatomy of a subject, and generating a masked image by applying the mask to acquired image data of the anatomy of the subject, including weighting the mask differently inside a region of interest (ROI) of the image than outside the ROI, the weighting inside ROI independent of the weighting outside the ROI. A user may be able to adjust a relative magnitude of subtraction inside and outside the ROI, and thus be able to visualize both vasculature and landmarks within the same image frame.

**SUMMARY OF THE INVENTION**

The invention is set out in the appended set of claims.

One of the problems to be solved by the embodiments described herein is to appropriately perform an emphasis processing of a target to be observed in an X-ray image. The problems to be solved by the embodiments described herein are not limited to the above-mentioned problem. The problem corresponding to each effect of each configuration shown in the embodiment described later can be another problem.

According to an embodiment, there is provided an apparatus according to claim 1.

The emphasis processing image generation unit may generate an emphasis processing image from an X-ray image by compositing the X-ray image and the extracted motion related component.

The motion suppression image generation unit may generate, as the motion suppression image, a mean value image or a median value image of two or more of the plurality of X-ray images.

When an X-ray irradiation is switched from ON to OFF and then returned to ON, the motion suppression image generation unit may update the motion suppression image based on the motion suppression image generated before switched to OFF and the X-ray image obtained after returned to ON.

When an irradiation field in X-ray imaging of the object is changed, the motion suppression image generation unit may generate the motion suppression image of the changed irradiation field such that the motion suppression image based on two or more of the plurality of time-series X-ray images imaged in the irradiation field before the change is used for the part of the changed irradiation field that overlaps with the irradiation field before the change.

The emphasis processing image generation unit may multiply the extracted motion related component by a factor to generate an intermediate emphasis image, and composite the intermediate emphasis image and the X-ray image to generate the emphasis processing image.

The emphasis processing image generation unit may narrow a window width of the emphasis processing image such that the target is emphasized.

The motion related components include movement derived from at least one of pulsation and respiration of the object and/or, when the target moves in the object, movement derived from movement of the target.

The emphasis processing image generation unit may generate the emphasis processing image by adding the difference image multiplied by a factor and the X-ray image corresponding to the difference image.

The emphasis processing image generation unit may narrow a window width of the emphasis processing image such that the target is emphasized.

According to an aspect, there is provided a medical image processing method according to claim 14.

This method can be applied to a medical image processing system including a client and a server. In this case, each step of the medical image processing method may be executed by either the client or the server.

**BRIEF DESCRIPTION OF THE DRAWINGS**

Fig. 1 is a block diagram showing an example of a medical image processing system 1 including a medical image processing apparatus according to the first embodiment.

Fig. 2 is a diagram for explaining conventional image processing for emphasizing a target to be observed such as contrast agents and catheters.

Fig. 3 is a diagram for explaining an example of a generation method of the emphasis processing image according to the first embodiment in the post process.

Fig. 4 is an explanatory diagram showing an example of an X-ray image In before processing and an emphasis processing image ESIn.

Fig. 5 is a diagram for explaining the emphasis process of the target to be observed when the X-ray irradiation field is widened and then the position of the irradiation field is changed.

Fig. 6 is an explanatory diagram showing an example of the emphasis processing of the contrast agent depicted in the X-ray image of the head.

Fig. 7 a flowchart showing an example of a procedure for appropriately performing emphasis processing of the target to be observed of the X-ray image.

Fig. 8 is an explanatory diagram showing an example of a data flow in the case of combining emphasis processing of the target of the X-ray image and multi-frequency processing.

Fig. 9 is a block diagram showing a configuration example of a medical image processing system including a medical image processing apparatus according to the second embodiment.

**DETAILED DESCRIPTION**

Hereinbelow, a description will be given of a medical image processing apparatus, an x-ray diagnostic apparatus, and a medical image processing method according to embodiments of the present invention with reference to the drawings.

(First embodiment)

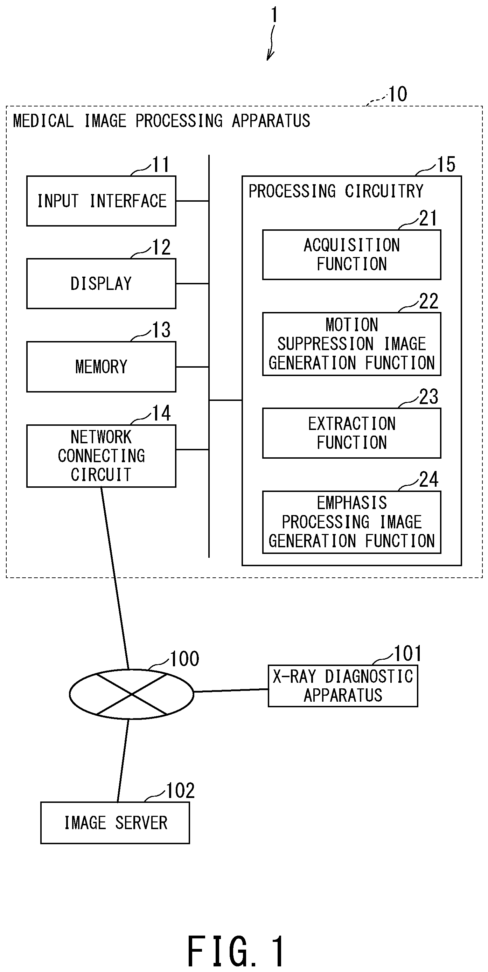

Fig. 1 is a block diagram showing an example of a medical image processing system 1 including a medical image processing apparatus 10 according to the first embodiment.

The medical image processing system 1 includes a medical image processing apparatus 10 and an X-ray diagnostic apparatus 101. The medical image processing apparatus 10 has an input interface 11, a display 12, a memory 13, a network connecting circuit 14, and processing circuitry 15. The medical image processing apparatus 10 is an example of a server.

The input interface 11 is composed of a general input device such as a trackball, a switch button, a mouse, a keyboard, and a numeric keypad, and outputs an operation input signal corresponding to a user's operation to the processing circuitry 15. The display 12 is configured by a general display device such as a liquid crystal display or an OLED (Organic Light Emitting Diode) display.

The memory 13 has a configuration including a recording medium readable by a processor, such as a RAM (Random Access Memory), a semiconductor memory device such as a flash memory, a hard disk, an optical disc, and the like, is used by the processing circuitry 15, and stores parameter data and other data. Part or all of the programs and data in the recording medium of the memory 13 may be downloaded by communication via the network 100, or may be provided to the memory 13 via a portable storage medium such as an optical disc.

The network connecting circuit 14 implements various information communication protocols according to the network 100. The network connecting circuit 14 connects the medical image processing apparatus 10 to other electric devices via the network 100 according to the various protocols. The network 100 refers to all information communication networks using telecommunications technology and includes wireless/wired LANs such as hospital LANs (Local Area Networks) and Internet networks, as well as telephone communication line networks, optical fiber communication networks, cable communications networks and satellite communication networks.

The medical image processing apparatus 10 is connected to the X-ray diagnostic apparatus 101 and the image server 102 via the network 100 so that data can be transmitted and received between them. The X-ray diagnostic apparatus 101 includes an X-ray angiography apparatus, mammography apparatus, an X-ray TV apparatus, and the like. The X-ray diagnostic apparatus 101 is an example of a client.

The processing circuitry 15 realizes functions of centrally controlling the medical image processing apparatus 10. In addition, the processing circuitry 15 is a processor that reads and executes the program stored in the memory 13 to execute processing for appropriately performing emphasis processing of the target to be observed depicted in the X-ray image.

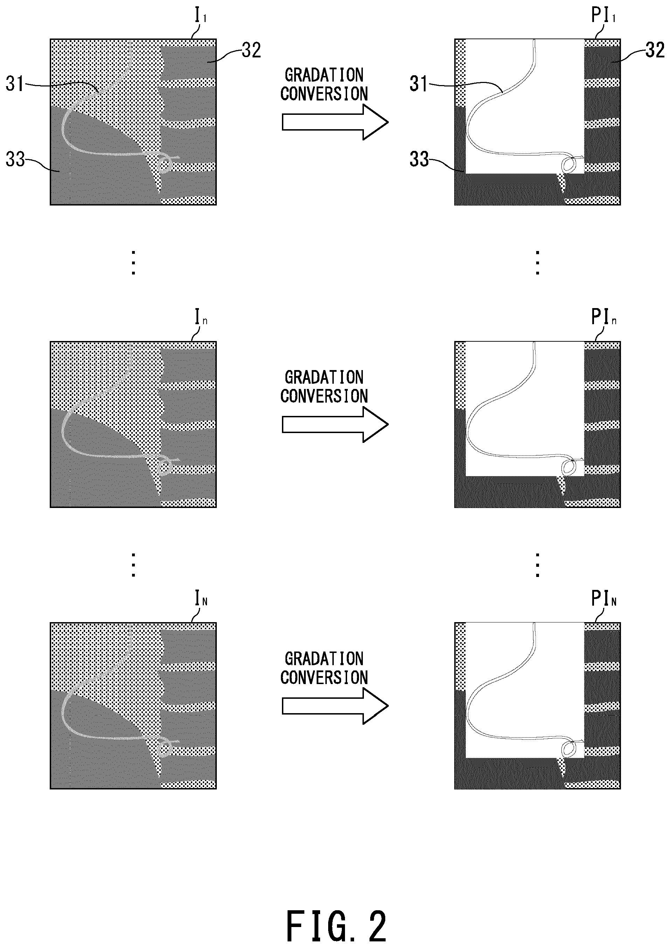

Fig. 2 is a diagram for explaining conventional image processing for emphasizing the target to be observed such as contrast agents and catheters.

Considering a case where the X-ray diagnostic apparatus 101 captures time-series N-frame X-ray images In (n=1, 2,..., N) of the object into which the catheter 31 is inserted. Conventionally, each X-ray image In is subjected to image processing such as background compression, signal enhancement, and gradation conversion to generate a processed image PIn, whereby the shade(shadow, contrast) of the catheter 31 which is the user's target to be observed is emphasized(enhanced) in the X-ray image. However, when non-target components other than the catheter 31 such as the bone 32 and the diaphragm 33 are depicted in the X-ray image In, shades of these non-target components are also emphasized by image processing such as gradation conversion. In this case, in the processed image PIn, non-target components such as the bone 32 and the diaphragm 33 impede the catheter 31 to make it difficult to observe the catheter 31.

Therefore, the processor of the processing circuitry 15 extracts the target from the X-ray image In and composites the intermediate emphasis image that emphasizes the extracted target with the X-ray image In to generate an image (hereinafter referred to as emphasis processing image) in which the target depicted in the X-ray image In is emphasized.

As shown in Fig. 1, the processor of the processing circuitry 15 implements the acquisition function 21, the motion suppression image generation function 22, the extraction function 23, and the emphasis processing image generation function 24. Each of these functions is stored in the memory 13 in the form of a program.

In the following description, the X-ray image subject to the emphasis processing of the target according to the present embodiment may be an X-ray fluoroscopic image, or an X-ray image imaged at a higher dose than the X-ray fluoroscopic image.

Further, in the present embodiment, an example in which each function 21-24 is implemented by the processing circuitry 15 of the medical image processing apparatus 10 will be described. However, Some or all of these functions 21-24 of the medical image processing apparatus 10 may be realized by an external device that is independent from the medical image processing apparatus 10 and has at least a processor and memory such as a hospital server, cloud console, workstation, X-ray diagnostic apparatus 101, connected to the network 100. Further, the medical image processing apparatus 10 may be configured by a plurality of information processing devices connected to each other via the network 100, and each function 21-24 may be appropriately distributed and implemented by the plurality of information processing devices.

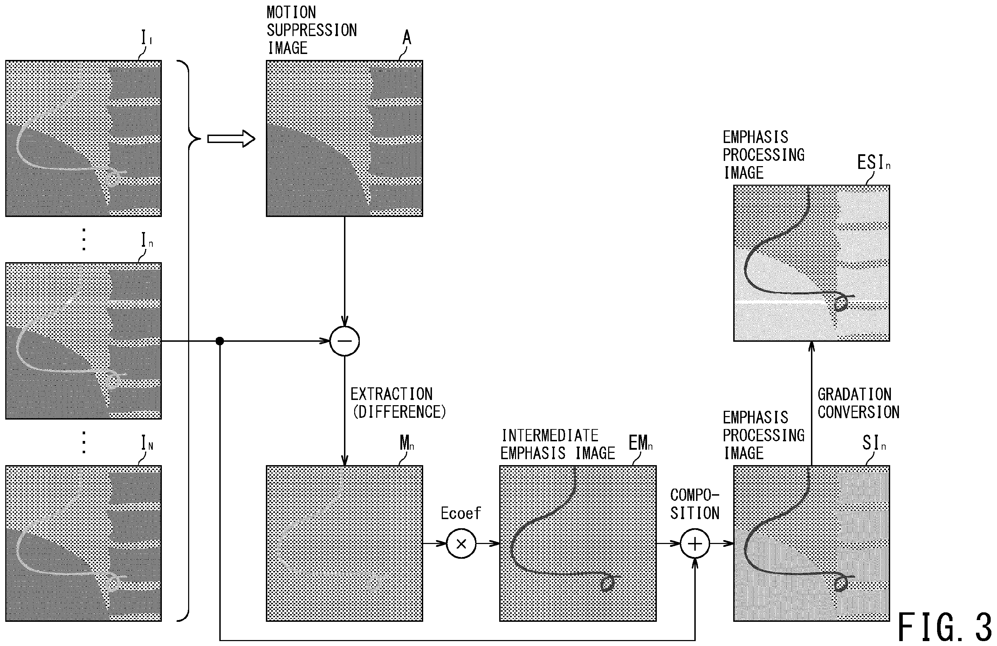

Next, the functions 21-24 will be described with reference to Fig. 3.

(Post process)

First, a case will be described in which all of a plurality of time-series X-ray images I1, I2,..., IN are acquired in the post process, and an emphasis processing image of the X-ray image In of these X-ray images is generated.

Fig. 3 is a diagram for explaining an example of a generation method of the emphasis processing image according to the first embodiment in the post process.

In the post process, the acquisition function 21 acquires the plurality of time-series X-ray images I1, I2,..., IN related to the object, which are obtained based on the X-ray imaging of the object performed by the X-ray diagnostic apparatus 101 (see the leftmost column in Fig. 3). The acquisition function 21 is an example of an acquisition unit. These X-ray images are acquired directly from the X-ray diagnostic apparatus 101 on which the X-ray imaging was performed, or indirectly via the image server 102.

In the post-process, the motion suppression image generation function 22 generates a motion suppression image in which motion related components are suppressed more than components that are stationary in at two or more of the plurality of time-series X-ray images, based on two or more of the plurality of time-series X-ray images I1, I2,..., IN. The motion suppression image generation function 22 is an example of a motion suppression image generation unit and a representative value image generation unit.

In the post-process, the motion suppression image A is generated based on two or more of the plurality of time-series X-ray images I1, I2,..., IN, and is an image (representative value image) each pixel of which has a representative value of pixel values of the two or more X-ray images.

For example, when the X-ray images I1, I2,..., IN are images of the heart of the object and the user's target to observe is the catheter 31, the catheter 31 moves greatly in the image by at least one of the pulsation and the respiratory movement of the object. In addition, when there is a body movement of the object, the entire image is displaced between frames. In this regard, by setting each pixel of the motion suppression image A as a representative value, the moving target is suppressed during imaging of the plurality of time-series X-ray images I1, I2,..., IN. A mean value or a median value can be used as the representative value, for example.

Fig. 3 shows an example in which the motion suppression image generation function 22 generates the mean value image A of all the X-ray images I1, I2,..., IN as the motion suppression image A (see two columns from the left in Fig. 3). In this case, the relationship between the pixel value A(i,j) of each pixel of the motion suppression image A (where i represents the x coordinate and j represents the y coordinate of each pixel) and the pixel value In(i,j) of each X-ray image In can be written as the following equation (1).

$$A(i,j) = \frac{1}{N}\sum_{n=1}^{N} I_n(i,j) \qquad\qquad (1)$$

Further, the motion suppression image A may be a moving average image in which the frame range and the total number of frames are fixed, where the range may be set from the frame to be processed to 20 frames before or may be set 10 frames before and after the frame to be processed. In this case, it is possible to reduce the influence of a specific frame in which a large change of state has occurred during imaging on the motion suppression image A. In this case, the frame range and the total number of frames used for generating the motion suppression image A may use the setting values stored in the memory 13 in advance, or can be set by the user via the input interface 11 and can be changed by the user. Also the frame range and the total number of frames used for generating the motion suppression image A may be set so as to correspond to the period of one heartbeat of the object, or the period of the number of heartbeats input by the user via the input interface 11, or the period of a predetermined number of heartbeats stored in the memory 13. In the real-time processing described later, it is preferable that the user can change the setting at any time regardless of whether the X-ray irradiation is on or off.

In the post process, the extraction function 23 extracts the user's observation target from the X-ray image In. The extraction function 23 is an example of an extraction unit and a subtraction image generation unit. Fig. 3 shows an example in which the target is the catheter 31 that is inserted into the heart and moves along with the pulsation. In this case, the extraction function 23 will generate the target image Mn by generating the difference image between the X-ray image In and the motion suppression image A (see the lower second column from the left in Fig. 3). In this case, the pixel value Mn(i,j) of each pixel of the target image Mn can be expressed by the following equation (2).

$$M_n(i,j) = I_n(i,j) - A(i,j) \qquad\qquad (2)$$

Note that the equation (2) shows an example in which a simple difference between the X-ray image In and the motion suppression image A is taken, but the difference may be taken after log-converting the X-ray image In and the motion suppression image A, or other subtraction methods may be used.

When the target itself moves within the object, such as a catheter 31 inserted into the heart or a contrast agent injected into the object, the extraction function 23 extracts information on the movement amount, the movement direction, and the like between the frames as the motion related component by extracting and comparing the linear shadows of the past frame (for example, the immediately preceding frame) and the processing target frame. In this case, it is not necessary to use the motion suppression image A for extracting the motion related component and generating the target image Mn, and thus the motion suppression image generation function 22 is not necessary.

In the post process, the emphasis processing image generation function 24 generates an emphasis processing image in which the target is emphasized in the X-ray image In based on the target image Mn, and displays it on the display 12.

Specifically, the emphasis processing image generation function 24 firstly determines, as shown in the following equation (3), multiplies each pixel of the target image Mn by a predetermined emphasis coefficient Ecoef. of a value of 0 or more (preferably 1 or more) to generate an intermediate emphasis image EMn. When the emphasis coefficient Ecoef. is larger than 1, the target is emphasized in the intermediate emphasized image EMn. When the emphasis coefficient Ecoef. is less than 1, the target is not emphasized in the intermediate emphasized image EMn, but the target can be emphasized in the emphasis processing image SIn discussed later.

$$EM_n(i,j) = M_n(i,j) \times Ecoef. \qquad (3)$$

In the example shown in Fig. 3, the catheter 31 extracted from the X-ray image In is emphasized (see the third column from the left in Fig. 3).

Note that the emphasis coefficient Ecoef. May be set values stored in the memory 13 in advance, or may be set by the user via the input interface 11 and changeable. In the real-time processing described later, it is preferable that the user can change the setting at any time regardless of whether the X-ray irradiation is on or off.

Further, the emphasis coefficient Ecoef. may be automatically set according to the parameters such as an SN ratio (signal-to-noise ratio) of a target X-ray image In, a CN ratio of a target (contrast-to-noise ratio), an X-ray condition including a tube voltage, a tube current, and a pulse width, a dose setting, the image processing setting, and the like. Even when the setting is automatically made in this way, the setting may be further changeable by the user.

Further, the generation process of the intermediate emphasis image EMn is not essential, and the target image Mn may be used instead of the intermediate emphasized image EMn to perform the generation process of the emphasis processing image SIn discussed later. Using the target image Mn instead of the intermediate emphasized image EMn has the same result as performing the emphasis process with the emphasis coefficient Ecoef. being 1.

The emphasis processing image generation function 24 then generates the emphasis processing image SIn by compositing the intermediate emphasis image EMn or the target image Mn and the original X-ray image In corresponding to the intermediate emphasis image EMn or the target image Mn. The composition may be simple addition or averaging, or weighted addition or weighted averaging. Fig. 3 shows an example of the case where the emphasis processing image SIn is generated by adding the intermediate emphasis image EMn and the X-ray image In (see the lower rightmost column in Fig. 3). In this case, the pixel value SIn(i,j) of each pixel of the emphasis processing image SIn can be expressed by the following equation (4).

$$SI_n(i,j) = I_n(i,j) + EM_n(i,j) \qquad (4)$$

The emphasis processing image SIn is an image generated by compositing the X-ray image In with the intermediate emphasis image EMn in which the target is emphasized. Therefore, the emphasis processing image SIn is an image in which the target is emphasized and the non-target components are suppressed as compared with the X-ray image In before emphasis processing or an image obtained by performing conventional image processing on the X-ray image In.

In generating the intermediate emphasis image EMn, the pixel value of the target is multiplied by the emphasis coefficient Ecoef. Therefore, when the contrast and noise of the target are set to be approximately the same as those of the original image, in the window processing (gradation processing) of the emphasis processing image Sin, the relationship between the window width WWpost and the window width WWpre of the original image satisfies the condition of the following equation (5).

$$WW_{post} = WW_{pre} \times (Ecoef. + 1) \qquad (5)$$

Even when the contrast and noise are similar to those of the original image, the image level difference for the non-target component is relatively small with respect to the target in the emphasis processing image SIn. Therefore, the emphasis processing image SIn is an image in which the target can be easily identified.

Further, the emphasis processing image generation function 24 may generate an image ESIn in which the contrast of target is further improved as compared with the emphasis processing image SIn by narrowing WWpost shown in equation (5) (see the uppermost column in the rightmost column of Fig. 3).

$$WW_{post} = WW_{pre} \times (Ecoef. + 1) \times \frac{1}{Cont_{up}} \qquad (6)$$

In equation (6), $Cont_{up}$ has a value of 1 or more. By narrowing the window width from WWpost shown in equation (5), only the contrast of the target can be improved without causing a large change in the visibility of the non-target components.

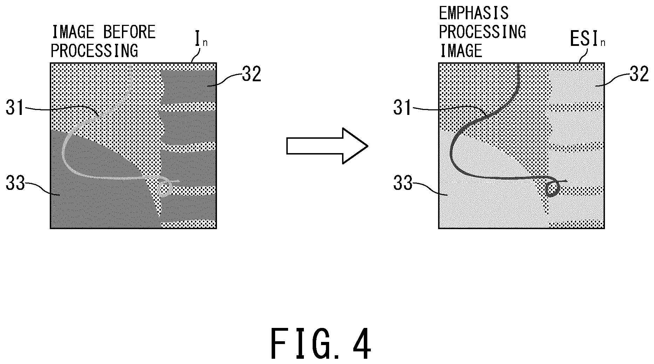

Fig. 4 is an explanatory diagram showing an example of the X-ray image In before processing and the emphasis processing image ESIn. As shown in Fig. 4, in the emphasis processing image ESIn, the catheter 31 of the X-ray image In is emphasized, while the bone 32 and diaphragm 33 which are the non-target components are suppressed.

As WWpost is narrowed, the contrast of the target is improved while the noise is also increased. Thus, the value of $Cont_{up}$ may be the setting value stored in the memory 13 in advance, or may be set by the user via the input interface 11 and changed by the user in consideration of contrast and noise.

Since the image level of the non-target components does not change significantly, the window center (window level) WC does not have to be changed when the non-target components occupy most of the image. Further, When an offset is added to the entire pixel value in a system in which a negative value cannot be used as the pixel value, the offset may be added to the window center.

(Real-time processing)

Next, real-time processing will be explained. Hereinafter, a case where the X-ray image In is newly acquired following a plurality of time-series X-ray images I1, I2,..., In-1, and the emphasis processing image of the newly acquired X-ray image In is generated.

In the real-time processing, the acquisition function 21 acquires the plurality of time-series X-ray images I1, I2,..., In of the object, which are obtained based on the X-ray imaging of the object by the X-ray diagnostic apparatus 101, from the X-ray diagnostic apparatus 101.

In the real-time processing, the motion suppression image generation function 22 generates the motion suppression image An in which the motion related component is suppressed based on two or more of the plurality of time-series X-ray images I1, I2,..., In. When the motion suppression image generation function 22 generates the mean value images An of all the X-ray images I1, I2,..., In acquired up to the present time in real-time processing as motion suppression image An, the relationship between the pixel value An(i,j) of each pixel of the motion suppression image An and the pixel value In(i,j) of each X-ray image In can be written as the following equation (7).

$$A_n(i,j) = \frac{n-1}{n} A_{n-1}(i,j) + \frac{1}{n} I_n(i,j) \qquad (7)$$

In this case, the motion suppression image An is recalculated and updated each time an X-ray image of a new frame is acquired.

Further, in this case, the extraction function 23 generates the target image Mn by generating a difference image between the X-ray image In and the motion suppression image An according to the following equation (8), for example.

$$M_n(i,j) = I_n(i,j) - A_n(i,j) \qquad (8)$$

The method for the emphasis processing image generation function 24 to generate the intermediate emphasis image EMn and the emphasis processing image SIn or ESIn does not differ between the post-process and the real-time processing, and thus the description thereof will be omitted.

The emphasis processing image SIn or ESIn generated by real-time processing have the same effect as those of post-process generated emotion processing image SIn or ESIn.

However, in the real-time processing, immediately after the start of the processing, the number of frames of the X-ray images used to generate the motion suppression image An is small, and hence, there is a case where the representative value image may not be the motion suppression image (image in which motion is suppressed), such as the case where afterimages of a plurality of catheters 31 are appeared in the representative value image.

Hence, in real-time processing, the emphasis processing image SIn or ESIn and the original image In may be displayed simultaneously. Further, after the processing is started, only the original image In is displayed up to a predetermined number of frames, while after the predetermined number of frames, the emphasis processing image SIn or ESIn and the original image In are simultaneously displayed or the original image In may be switched to the emphasis processing image SIn or ESIn. Further, even in real-time processing, only the emphasis processing image SIn or ESIn may be displayed immediately after the start of processing. These display methods may be selectable by the user.

Immediately after the start of real-time processing, as described above, motion related components may remain in the representative value image. Therefore, in the generation of the intermediate emphasis image EMn, the emphasis coefficient Ecoef. may be changed to be gradually increased.

When there is no change in the irradiation field, motion suppression image A may be taken over and processing may continue regardless of whether X-ray irradiation is on or off. For example, X-ray irradiation is repeatedly turned on and off in the X-ray fluoroscopic imaging. In real-time processing, by taking over the motion suppression image An and continuing processing regardless of whether the X-ray irradiation is turned on or off, it is possible to avoid the inconvenience caused by the insufficient number of frames immediately after the X-ray irradiation is turned on again. Further, the motion suppression image A created in the post process may be used in the subsequent real time processing.

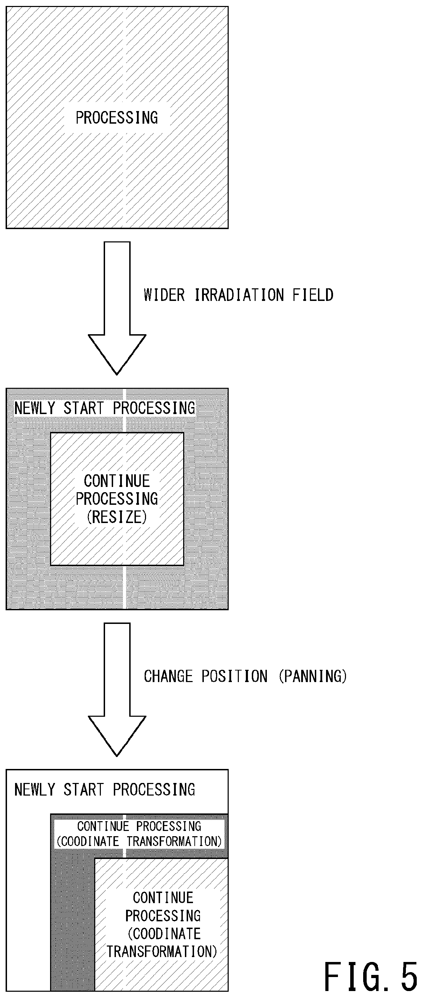

Fig. 5 is a diagram for explaining the emphasis process of the target to be observed when the X-ray irradiation field is widened and then the position of the irradiation field is changed.

Even when the irradiation field is changed, the motion suppression image A generated before the change of the irradiation field can be used by obtaining the information on the irradiation field before and after the change from the X-ray diagnostic apparatus 101. When the irradiation field in the X-ray imaging of object is changed, the motion suppression image of the changed irradiation field can be generated such that the motion suppression image of the irradiation field before change, which is scaled (resized) or coordinate converted, is used as the part of the motion suppression image corresponding to the part of the irradiation field after the change that overlaps the irradiation field before the change.

Further, the emphasis processing of the target to be observed according to the present embodiment can also be applied to the X-ray image of the part that is not affected by the pulsation or the respiratory motion.

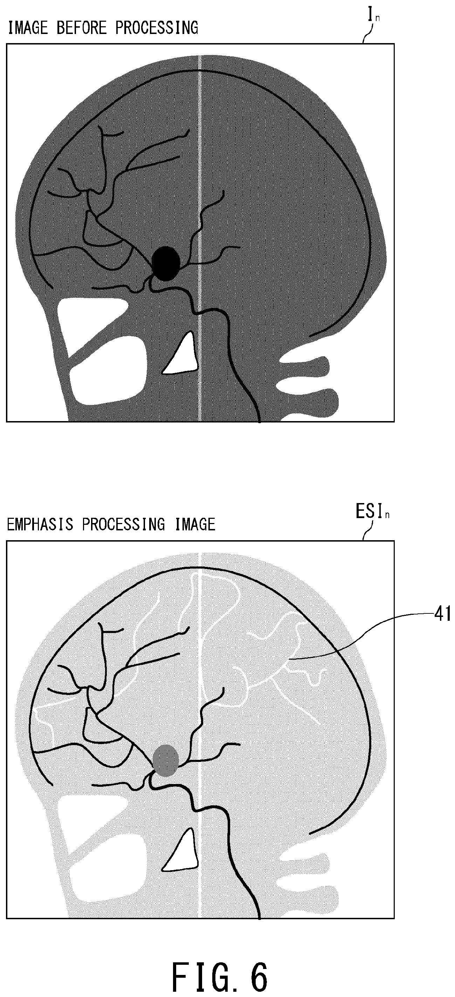

Fig. 6 is an explanatory diagram showing an example of the emphasis processing of the contrast agent depicted in the X-ray image of the head. Fig. 6 shows an example in which the target to be observed by the user is a contrast agent.

For example, considering a case where the motion suppression image generation function 22 generates the mean value image A of all X-ray images I1, I2,..., IN as motion suppression image A in the post process according to equation (1). Also in this case, since the motion suppression image A is the representative value image, the motion suppression image A is an image in which the influence of body movement is suppressed. Therefore, even if the target image Mn is generated by the extraction function 23 by the difference processing of equation (2), for example, an artifact due to misregistration does not occur.

Further, in this case, the motion suppression image A is generated by using the X-ray image In+1 and its subsequent X-ray images in which the contrast agent has already reached the blood vessel while the contrast agent has not reached the target X-ray image In. Meanwhile, the head does not move significantly except for body movement. Thus, the influence of body movement is suppressed, while the contrast agent is not suppressed even in the motion suppression image A. Therefore, the route of contrast agent (route of blood vessel) is drawn in a wide range in the motion suppression image A. Hence, as shown in Fig. 6, even in the emphasis processing image ESIn in which the flow of the contrast medium is emphasized as a motion, the route 41 of the blood vessel which is not dyed in the X-ray image In (but is dyed in its subsequent frames) can be depicted in a wide range. Therefore, the user can visually recognize the route of the blood vessel by checking the emphasis processing image ESIn, and thus can easily grasp the flow of the contrast agent.

Next, an example of operations of the medical image processing apparatus, the X-ray diagnostic apparatus, and the medical image processing system according to the present embodiment will be described.

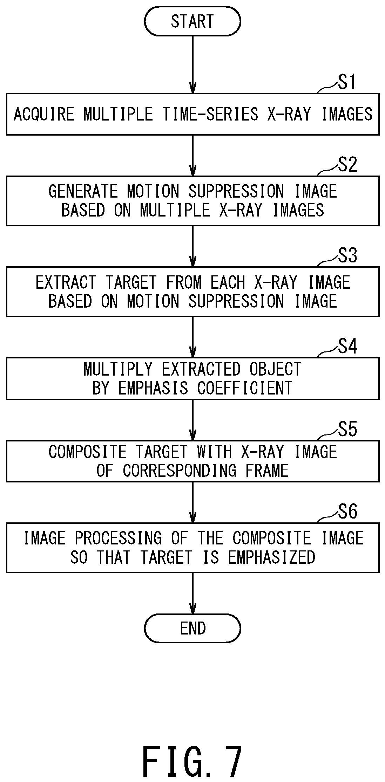

Fig. 7 a flowchart showing an example of a procedure for appropriately performing emphasis processing of the target to be observed of the X-ray image. Fig. 7 shows an example of processing in the post process.

First, in step S1, the acquisition function 21 obtains a plurality of time-series X-ray images I1, I2,..., IN of the object, which are obtained based on the X-ray imaging of the object by the X-ray diagnostic apparatus 101.

Next, in step S2, the motion suppression image generation function 22 generates the motion suppression image A in which motion related components are suppressed based on two or more of the plurality of time-series X-ray images I1, I2,..., IN,

Next, in step S3, the extraction function 23 extracts the user's target from the X-ray image In and generates the target image Mn.

Next, in step S4, the emphasis processing image generation function 24 generates the intermediate emphasis image EMn emphasizing the target by multiplying each pixel of the target image Mn by the predetermined emphasis coefficient Ecoef. having a value of 0 or more.

Next, in step S5, the emphasis processing image generation function 24 generates the emphasis processing image SIn by compositing the intermediate emphasis image EMn and the corresponding original X-ray image In.

Next, in step S6, the emphasis processing image generation function 24 generates the emphasis processing image ESIn in which the contrast of the target to be observed is further improved from the emphasis processing image Sin by narrowing the window width WW of the emphasis processing image Sin from WWpost shown in equation (5).

With the above procedure, it is possible to appropriately perform the emphasis processing of the target to be observed in the X-ray image.

According to the medical image processing system 1 including the medical image processing apparatus 10 according to the present embodiment, the target is extracted from the X-ray image In, and the intermediate emphasis image EMn in which the extracted target is emphasized is composited with the X-ray image In, whereby it is possible to generate the emphasis processing image SIn in which the target depicted on the X-ray image In is emphasized.

Target is emphasized and non-target is suppressed in emphasis processing image SIn and emphasis processing image ESIn that is further gradation converted (see Fig. 4). Therefore, the user can observe the target accurately and in a short time even when the contrast agent or the catheter 31 that is inserted into the heart and is constantly moving due to the pulsation is the target. For example, since the contrast agent can be emphasized in the emphasis processing image, even a small amount of the contrast agent can be clearly observed in the emphasis processing image. Therefore, the amount of the contrast agent administered into the object can be reduced. Further, in the case of real-time processing, it becomes possible to shorten the X-ray imaging time, and it is possible to reduce the exposure dose of the object.

Further, the emphasis processing image generation function 24 may combine the processing using the spatial frequency with the emphasis processing of the target of the X-ray image. Examples of processing using spatial frequencies include multi-objective frequency processing and wavelet transform.

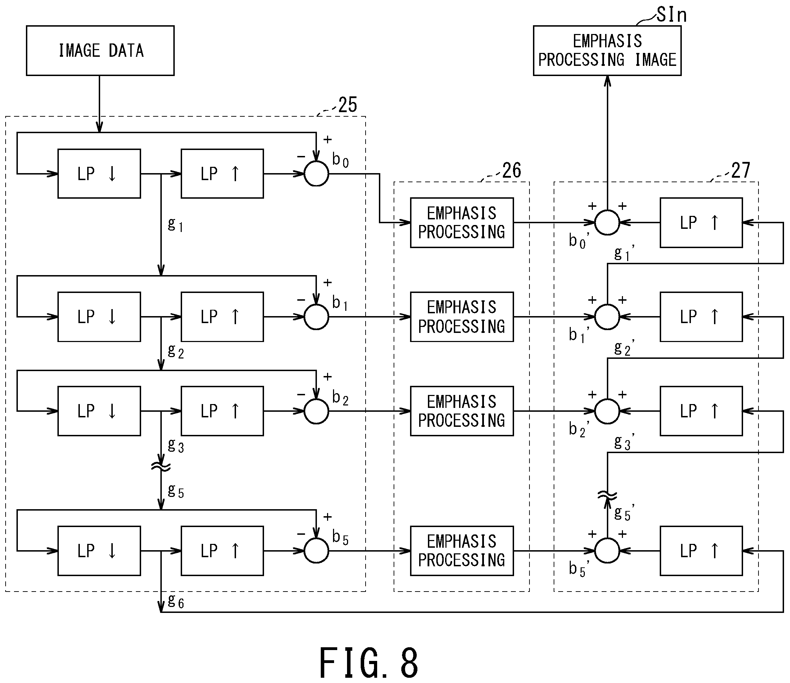

Fig. 8 is an explanatory diagram showing an example of a data flow in the case of combining emphasis processing of the target of the X-ray image and multi-frequency processing.

When the target emphasis processing of the X-ray image and the multi-frequency processing are combined, the processor of the processing circuitry 15 further includes the frequency band data generation function 25, the emphasis processing function 26, and the frequency band data synthesis function 27. These configurations 25-27 may be realized by software, hardware alone or a mixture of hardware and software. The frequency band data synthesis function 27 may be included in the emphasis processing image generation function 24.

The frequency band data generation function 25 converts the X-ray image In into a plurality of frequency band data. Specifically, the frequency band data generation function 25 generates background data (for example, one background data) and the plurality of frequency band data each including a predetermined frequency band from the X-ray image In. For example, the frequency band data generation function 25 generates the plurality of frequency band data each including different frequency band by performing LPF (Low Pass Filter) processing stepwise and taking a difference from the LPF processed image of one step before.

For example, the frequency band data generation function 25 first extracts low frequency data by performing LPF processing on the X-ray image In at the first stage LP↓. Here, the frequency band data generation function 25 may execute the downsampling process at LP↓ in order to speed up the subsequent processes. In this case, the frequency band data generation function 25 thins out pixels every other pixel in the horizontal direction from the low frequency data and then thins out pixels every other pixel in the vertical direction from the low frequency data to generate the low resolution image data g1 with the image size reduced to 1/4.

Then, the frequency band data generation function 25 sends the low resolution image data g1 to the second stage, and executes the upsampling process and the LPF process at LP ↑, thereby performing the LPF process with the same size as the X-ray image In to generate low frequency data. For example, the frequency band data generation function 25 first complements the low resolution image data g1 with "0" every other pixel in the horizontal direction and then complements "0" with every other pixel in the vertical direction, and the LPF processing in which each element of the LPF is multiplied by 4 is executed. Then, the frequency band data generation function 25 generates the frequency band data b0 by subtracting the X-ray image In and the low frequency data for each pixel by the adder. A Gaussian filter of about 5×5 can be used for the LPF treatment by the frequency band data generation function 25.

The frequency band data generation function 25 executes the processing of the second and subsequent stages in the same manner as the processing of the first stage described above. The image data to be processed in each stage is the low resolution image data generated in the preceding stage. That is, the image data to be processed in the second stage is the low-resolution image data g1, and the low-resolution image data g2 to g5 generated in each stage is the image data to be processed in the respective subsequent stages. Then, the frequency band data generation function 25 uses the low resolution image data g2 to g5 in each stage to generate the frequency band data b1 to b5 as in the first stage. As such, the frequency band data generation function 25 generates the stepwise frequency band data of the X-ray image In and the background data g6 in which the contained information is only the background.

Further, the frequency band data generation function 25 generates stepwise frequency band data of the motion suppression image A by performing the same processing as the X-ray image In on the motion suppression image A. The background data g6 may not be generated for either the X-ray image In or the motion suppression image A.

Although Fig. 8 shows the case where the frequency band data generation function 25 executes the process of 6 stages, the embodiment is not limited to the case and the process can be performed in any number of stages.

The emphasis processing function 26 controls the extraction function 23 and takes the difference between the frequency band data of the X-ray image In and the frequency band data of the motion suppression image A between the corresponding frequency bands to generate difference image data for each frequency band (hereinafter referred to as band difference image data).

The ratio of incoming signals of interest (the ratio of incoming signals of moving components that are target to be observed) is considered to differ for each spatial frequency band. Therefore, the emphasis processing function 26 obtains the ratio of target included in each of the plurality of band difference image data, and based on the obtained ratio of target, assigns the emphasis coefficient Ecoef. to each of the plurality of band difference image data independently of each other.

Further, the emphasis processing function 26 controls the emphasis processing image generation function 24, and generates the intermediate emphasis image data (hereinafter, referred to as band intermediate emphasis image data) for each of the frequency bands by using the emphasis coefficient Ecoef. assigned to each of the plurality of band difference image data. In addition, the emphasis processing function 26 adds the band intermediate emphasis image data and the frequency band data of the X-ray image In between the corresponding frequency bands to generate the emphasis processing image data for each frequency band (hereinafter, the band emphasis processing image data). Further, image processing such as background compression, signal enhancement, and gradation conversion may be performed on each of the band emphasis processing image data.

The frequency band data synthesis function 27 synthesizes a plurality of the band emphasis processing image data to generate an emphasis processing image SIn. Specifically, the frequency band data synthesizing function 27 sequentially synthesizes the background data g6 and the band emphasis processing image data b0' to b5' to generate the emphasis processing image SIn having the same size as the X-ray image In. For example, the frequency band data synthesizing function 27 performs the upsampling process on the background data g6 at the first stage (lowermost stage in the figure) LPT (by complementing "0" for every other pixel in horizontal direction and then complementing "0" for every other pixel in the vertical direction) and performing the same LPF processing as the upsampling processing of the frequency band data generation function 25, whereby the background data g6 has the same size as the band emphasis processing image data b5'. Then, the frequency band data synthesizing function 27 generates the added data g5' by adding the same size of the band emphasis processing image data b5' and the background data g6 for each pixel by the adder.

The frequency band data synthesizing function 27 executes the above-described upsampling process and LPF process on the generated added data g5' to make the added data g5' the same size as the band emphasis processing image data b4'. The frequency band data synthesizing function 27 then adds the added data g5' with the processing image data b4' to generate added data g4'. The frequency band data synthesis function 27 similarly generates the emphasis processing image SIn in which the target is emphasized in the same size as the X-ray image In by increasing the size of the added data and performing addition with the band emphasis processing image data sequentially. The frequency band data synthesis function 27 may be one function included in the emphasis processing image generation function 24.

In this way, by combining the emphasis processing and the multi-frequency processing shown in Fig. 7, it is possible to apply an appropriate emphasis coefficient Ecoef. for each frequency band data. Enhancement coefficient Ecoef. has different appropriate value in each frequency band. Therefore, by combining the emphasis processing and the multi-frequency processing, it is possible to more selectively emphasize the signal of interest in the emphasis processing image SIn and the emphasis processing image ESIn obtained by further gradation conversion of the emphasis processing image SIn. Therefore, the target is further emphasized while the non-target components can be suppressed, as compared with the case where only the emphasis processing is applied.

(Second embodiment)

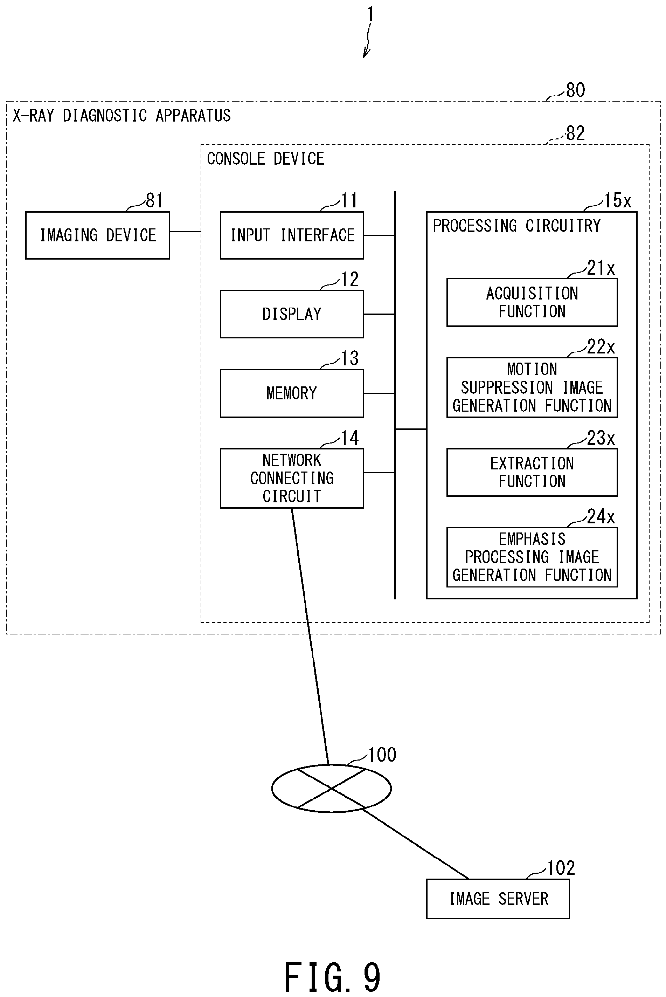

Fig. 9 is a block diagram showing a configuration example of the medical image processing system 1 including the medical image processing apparatus 10 according to the second embodiment.

The X-ray diagnostic apparatus 80 includes the imaging device 81 that performs imaging of time-sequential N-frame X-ray images I1, I2,..., IN concerning the object, and the console device 82 as an example of the medical image processing apparatus 10. The X-ray diagnostic apparatus 80 shown in the second embodiment differs from the medical image processing apparatus 10 shown in the first embodiment in that a plurality of time-series X-ray images I1, I2,..., IN generated by itself by X-ray imaging of the object an be used. Since other configurations and operations are substantially the same as those of the medical image processing apparatus 10 shown in Fig. 1, the same configurations are denoted by the same reference numerals and description thereof will be omitted.

The imaging device 81 is composed of, for example, an imaging system of an X-ray angiography apparatus, has an imaging system such as an X-ray tube and an X-ray detector for capturing an X-ray image of the object placed on a top plate. The imaging device 81 provides the console device 82 with the plurality of time-series projection data regarding the object obtained by imaging the object.

The acquisition function 21x of the processing circuitry 15x acquires the plurality of time-series X-ray images I1, I2,..., IN regarding the object from the reconstruction function 20. The motion suppression image generation function 22x generates the motion suppression image A in which the motion related component is suppressed based on two or more of the plurality of time-series X-ray images I1, I2,..., IN. The extraction function 23x extracts the user's target from the X-ray image In and generates the target image Mn. The emphasis processing image generation function 24x generates the emphasis processing image SIn or ESIn in which the target is emphasized in the X-ray image In based on the target image Mn, and displays it on the display of the console device 82.

As in the medical image processing system 1 according to the first embodiment, the medical image processing system 1 including the X-ray diagnostic apparatus 80 according to the second embodiment also extracts the target from the X-ray image In, composites the intermediate emphasis image EMn in which the extracted target is emphasized with the X-ray image In, thereby generating the emphasis processing image SIn in which the target depicted on the X-ray image In is emphasized.

According to at least one of the above-described embodiments, an emphasis processing of a target to be observed in an X-ray image can be appropriately performed.

The processing circuitry in the above-described embodiments is an example of the processing circuitry described in the claims. In addition, the term "processor" used in the explanation in the above-described embodiments, for instance, refer to circuitry such as dedicated or general purpose CPUs (Central Processing Units), dedicated or general-purpose GPUs (Graphics Processing Units), or ASICs (Application Specific Integrated Circuits), programmable logic devices including SPLDs (Simple Programmable Logic Devices), CPLDs (Complex Programmable Logic Devices), and FPGAs (Field Programmable Gate Arrays), and the like. The processor implements various types of functions by reading out and executing programs stored in the memory circuitry.

In addition, instead of storing programs in the memory circuitry, the programs may be directly incorporated into the circuitry of the processor. In this case, the processor implements each function by reading out and executing each program incorporated in its own circuitry. Moreover, although in the above-described embodiments an example is shown in which the processing circuitry configured of a single processor implements every function, the processing circuitry may be configured by combining plural processors independent of each other so that each processor implements each function of the processing circuitry by executing corresponding program. When a plurality of processors are provided for the processing circuitry, the memory medium for storing programs may be individually provided for each processor, or one memory circuitry may collectively store programs corresponding to all the functions of the processors.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the invention as claimed. The accompanying claims are intended to cover such forms or modifications as would fall within the scope of the invention as claimed.

**Claims**

1. An X-ray diagnostic apparatus comprising:
   an acquisition unit (21) configured to acquire a plurality of time-series X-ray images ($I_n$) of an object within which a moving target is provided, the apparatus **characterized by**:

   a motion suppression image generation unit (22) configured to generate, based on two or more of the plurality of time-series X-ray images ($I_n$), a motion suppression image A in which motion related components regarding the moving target are suppressed more than components in the two or more of the plurality of time-series X-ray images ($I_n$), wherein, in the motion suppression image (A), each pixel has a representative value of pixel values of the two or more X-ray images ($I_n$) so that the moving target is suppressed;
   **characterized in that** the apparatus further comprises a subtraction image generation unit (23) configured to generate a difference image ($M_n$) by difference processing between the motion suppression image (A) and one of the time-series X-ray images ($I_n$); and
   an emphasis processing image generation unit (24) configured to generate an emphasis processing image ($SI_n$) in which the moving target is emphasized in the one of the time-series X-ray images ($I_n$) based on the generated difference image ($M_n$), a size of the emphasis processing image ($SI_n$) being the same as that of the motion suppression image (A).

2. The X-ray diagnostic apparatus according to claim 1, wherein the emphasis processing image generation unit (24) is configured to generate an emphasis processing image from an X-ray image by compositing the X-ray image and the difference image.

3. The X-ray diagnostic apparatus according to claim 1 or 2,
   further comprising an extraction unit (23) is configured to extract motion related components in each of the plurality of X-ray images by subtracting the motion suppression image from the each of the plurality of X-ray images.

4. The X-ray diagnostic apparatus according to any preceding claim, the motion suppression image generation unit (22) is configured to generate, as the motion suppression image, a mean value image or a median value image of two or more of the plurality of X-ray images.

5. The X-ray diagnostic apparatus according to any preceding claim, wherein

when an X-ray irradiation is switched from ON to OFF and then returned to ON, the motion suppression image generation unit (22) is configured to update the motion suppression image based on the motion suppression image generated before switched to OFF and the X-ray image obtained after returned to ON.

6. The X-ray diagnostic apparatus according to any preceding claims, wherein
when an irradiation field in X-ray imaging of the object is changed, the motion suppression image generation unit (22) is configured to generate the motion suppression image of the changed irradiation field such that the motion suppression image based on two or more of the plurality of time-series X-ray images imaged in the irradiation field before the change is used for the part of the changed irradiation field that overlaps with the irradiation field before the change.

7. The X-ray diagnostic apparatus according to claim 2, wherein the emphasis processing image generation unit (24) is adapted to multiply the extracted motion related component by a factor to generate an intermediate emphasis image, and composite the intermediate emphasis image and the X-ray image to generate the emphasis processing image.

8. The X-ray diagnostic apparatus according to claim 7, further comprising:
an extraction unit (23) is configured to extract motion related components in each of the plurality of X-ray images by subtracting the motion suppression image from the each of the plurality of X-ray images; and
an emphasis processing unit configured to:

convert the X-ray image into a plurality of frequency band data;
assign the emphasis coefficient to each of the plurality of frequency band data in accordance with a ratio of the extracted motion related component by cooperating with the extraction unit (23) and the emphasis processing image generation unit (24); and
generate image data of the intermediate emphasis image for the each of the plurality of frequency bands based on the assigned emphasis coefficient,
wherein the emphasis processing image generation unit (24) is configured to generate the emphasis processing image based on the image data of the intermediate emphasis image for the each of the plurality of frequency bands.

9. The X-ray diagnostic apparatus according to any one of claims 1 to 8, wherein the emphasis processing image (ESIn) generation unit (24) is adapted to narrow a window width of the emphasis processing image (SIn) such that the target is emphasized.

10. The X-ray diagnostic apparatus according to any one of claims 1 to 9, wherein the motion related components include movement derived from at least one of pulsation and respiration of the object and/or, when the target moves in the object, movement derived from movement of the target.

11. The X-ray diagnostic apparatus according to any one of claims 1 to 10, wherein:
the motion suppression image generation unit (22) is configured to generate a representative value image as the motion suppression image in which each pixel has a representative value of pixel values in the two or more X-ray images, based on the two or more X-ray images of the plurality of time-series X-ray images and the emphasis processing image generation unit (24) is configured to generate the emphasis processing image of the target depicted in the difference image by adding the generated difference image and the X-ray image corresponding to the difference image.

12. The X-ray diagnostic apparatus according to claim 11, wherein the emphasis processing image generation unit (24) is configured to generate the emphasis processing image by adding the difference image multiplied by a factor and the X-ray image corresponding to the difference image.

13. The X-ray diagnostic apparatus according to any one of claims 11 to 12, wherein the emphasis processing image generation unit (24) is configured to narrow a window width of the emphasis processing image such that the target is emphasized.

14. A medical image processing method comprising:
acquiring a plurality of time-series X-ray images of an object within which a moving target is provided, the method **characterized by**:

generating, based on two or more of the plurality of time-series X-ray images, a motion suppression image in which motion related components regarding the moving target are suppressed more than components in the two or more of the plurality of time-series X-ray images, wherein, in the motion suppression image, each pixel has a representative value of pixel values of the two or more X-ray images so that the moving target is suppressed; generating a difference image by difference processing between the motion suppression image and one of the time-series X-ray images; and

generating an emphasis processing image in which the moving target is emphasized in the one of the time-series X-ray images based on the generated difference image, a size of the emphasis processing image being the same as that of the motion suppression image.

15. The medical image processing method according to claim 14, wherein:

the acquiring the X-ray images is executed by any one of a client and a server both of which a medical image processing system includes;

the extracting the motion related component is executed by any one of the client and the server; and

the generating the emphasis processing image is executed by any one of the client and the server.

**Patentansprüche**

1. Röntgendiagnostikeinrichtung, umfassend:

eine Erfassungseinheit (21), die konfiguriert ist, um eine Vielzahl von Zeitreihen-Röntgenbildern ($I_n$) eines Objekts zu erfassen, innerhalb dessen ein sich bewegendes Ziel bereitgestellt ist, wobei die Einrichtung **gekennzeichnet ist durch**:

eine Bewegungsunterdrückungsbildgenerierungseinheit (22), die konfiguriert ist, um auf der Grundlage von zwei oder mehr der Vielzahl von Zeitreihen-Röntgenbildern ($I_n$) ein Bewegungsunterdrückungsbild A zu generieren, in dem bewegungsbezogene Komponenten in Bezug auf das sich bewegende Ziel stärker unterdrückt werden als Komponenten in den zwei oder mehr der Vielzahl von Zeitreihen-Röntgenbildern ($I_n$), wobei in dem Bewegungs-unterdrückungsbild (A) jedes Pixel einen repräsentativen Wert der Pixelwerte der zwei oder mehr Röntgenbilder ($I_n$) aufweist, sodass das sich bewegende Ziel unterdrückt wird;

**dadurch gekennzeichnet, dass** die Einrichtung weiter umfasst

eine Subtraktionsbildgenerierungseinheit (23), die konfiguriert ist, um **durch** Differenzverarbeitung zwischen dem Bewegungsunterdrückungsbild (A) und einem der Zeitreihen-Röntgenbilder ($I_n$) ein Differenzbild ($M_n$) zu generieren; und

eine Hervorhebungsverarbeitungsbildgenerierungseinheit (24), die konfiguriert ist, um ein Hervorhebungsver-arbeitungsbild ($SI_n$) zu generieren, in dem das sich bewegende Ziel in dem einem der Zeitreihen-Röntgenbilder ($I_n$) auf der Grundlage des generierten Differenzbildes ($M_n$) hervorgehoben wird, wobei eine Größe des Hervor-hebungsverarbeitungsbildes ($SI_n$) die gleiche ist wie die des Bewegungsunterdrückungsbildes (A).

2. Röntgendiagnostikeinrichtung nach Anspruch 1, wobei die Hervorhebungsverarbeitungsbildgenerierungseinheit (24) konfiguriert ist, um aus einem Röntgenbild ein Hervorhebungsverarbeitungsbild zu generieren, indem das Röntgenbild und das Differenzbild zusammengesetzt werden.

3. Röntgendiagnostikeinrichtung nach Anspruch 1 oder 2, weiter umfassend eine Extraktionseinheit (23), die konfiguriert ist, um in jedem der Vielzahl von Röntgenbildern bewegungsbezogene Komponenten zu extrahieren, indem das Bewegungsunterdrückungsbild von jedem der Vielzahl von Röntgenbildern subtrahiert wird.

4. Röntgendiagnostikeinrichtung nach einem vorstehenden Anspruch, wobei die Bewegungsunterdrückungsbildgenerierungseinheit (22) konfiguriert ist, um als das Bewegungsunterdrückungsbild ein Mittelwertbild oder ein Medianwertbild aus zwei oder mehr der Vielzahl von Röntgenbildern zu generieren.

5. Röntgendiagnostikeinrichtung nach einem vorstehenden Anspruch, wobei, wenn eine Röntgenbestrahlung von EIN auf AUS geschaltet und dann auf EIN zurückgebracht wird, die Bewegungsunterdrückungsbildgenerierungseinheit (22) konfiguriert ist, um das Bewegungsunterdrückungsbild auf der Grundlage des vor dem Schalten auf AUS generierten Bewegungsunterdrückungsbildes und des nach dem Zurückbringen auf EIN erhaltenen Röntgenbildes zu aktualisieren.

**6.** Röntgendiagnostikeinrichtung nach einem der vorstehenden Ansprüche, wobei, wenn das Bestrahlungsfeld bei der Röntgenbildgebung des Objekts geändert wird, die Bewegungsunterdrückungsbildgenerierungseinheit (22) konfiguriert ist, um das Bewegungsunterdrückungsbild des geänderten Bestrahlungsfeldes derart zu generieren, dass das Bewegungsunterdrückungsbild auf der Grundlage von zwei oder mehr der Vielzahl von Zeitreihen-Röntgenbildern, die vor der Änderung in dem Bestrahlungsfeld aufgenommen wurden, für den Teil des geänderten Bestrahlungsfeldes verwendet wird, der mit dem Bestrahlungsfeld vor der Änderung überlappt.

**7.** Röntgendiagnostikeinrichtung nach Anspruch 2, wobei die Hervorhebungsverarbeitungsbildgenerierungseinheit (24) angepasst ist, um die extrahierte bewegungsbezogene Komponente mit einem Faktor zu multiplizieren, um ein Zwischenhervorhebungsbild zu generieren und das Zwischenhervorhebungsbild und das Röntgenbild zusammenzusetzen, um das Hervorhebungsverarbeitungsbild zu generieren.

**8.** Röntgendiagnostikeinrichtung nach Anspruch 7, weiter umfassend:

eine Extraktionseinheit (23), die konfiguriert ist, um bewegungsbezogene Komponenten in jedem der Vielzahl von Röntgenbildern zu extrahieren, indem das Bewegungsunterdrückungsbild von jedem der Vielzahl von Röntgenbildern subtrahiert wird; und
eine Hervorhebungsverarbeitungseinheit, die konfiguriert ist zum:

Umwandeln des Röntgenbildes in eine Vielzahl von Frequenzbanddaten;
Zuweisen des Hervorhebungskoeffizienten an jede der Vielzahl von Frequenzbanddaten gemäß einem Verhältnis der extrahierten bewegungsbezogenen Komponente durch Zusammenarbeit mit der Extraktionseinheit (23) und der Hervorhebungsverarbeitungsbildgenerierungseinheit (24); und
Generieren von Bilddaten des Zwischenhervorhebungsbildes für jedes der Vielzahl von Frequenzbändern auf der Grundlage des zugewiesenen Hervorhebungskoeffizienten,
wobei die Hervorhebungsverarbeitungsbildgenerierungseinheit (24) konfiguriert ist, um das Hervorhebungsverarbeitungsbild auf der Grundlage der Bilddaten des Zwischenhervorhebungsbildes für jedes der Vielzahl von Frequenzbändern zu generieren.

**9.** Röntgendiagnostikeinrichtung nach einem der Ansprüche 1 bis 8, wobei die Erzeugungseinheit (24) für das Hervorhebungsverarbeitungsbild (ESIn) angepasst ist, um eine Fensterbreite des Hervorhebungsverarbeitungsbildes (SIn) derart zu verengen, dass das Ziel hervorgehoben wird.

**10.** Röntgendiagnostikeinrichtung nach einem der Ansprüche 1 bis 9, wobei die bewegungsbezogenen Komponenten Bewegung einschließen, die von mindestens einem stammt von Pulsschlag und Atmung des Objekts und/oder, wenn sich das Ziel in dem Objekt bewegt, Bewegung, die von Bewegung des Ziels stammt.

**11.** Röntgendiagnostikeinrichtung nach einem der Ansprüche 1 bis 10, wobei:
die Bewegungsunterdrückungsbildgenerierungseinheit (22) konfiguriert ist, um ein repräsentatives Wertbild als das Bewegungsunterdrückungsbild, in dem jedes Pixel einen repräsentativen Wert der Pixelwerte in den zwei oder mehr Röntgenbildern aufweist, auf der Grundlage von den zwei oder mehr Röntgenbildern der Vielzahl von Zeitreihen-Röntgenbildern zu generieren, und die Hervorhebungsverarbeitungsbildgenerierungseinheit (24) konfiguriert ist, um das Hervorhebungsverarbeitungsbild des in dem Differenzbild dargestellten Ziels durch Hinzufügen des generierten Differenzbildes und des Röntgenbildes, das dem Differenzbild entspricht, zu generieren.

**12.** Röntgendiagnostikeinrichtung nach Anspruch 11, wobei die Hervorhebungsverarbeitungsbildgenerierungseinheit (24) konfiguriert ist, um das Hervorhebungsverarbeitungsbild durch Hinzufügen des mit einem Faktor multiplizierten Differenzbildes und des dem Differenzbild entsprechenden Röntgenbildes zu generieren.

**13.** Röntgendiagnostikeinrichtung nach einem der Ansprüche 11 oder 12, wobei die Hervorhebungsverarbeitungsbildgenerierungseinheit (24) konfiguriert ist, um eine Fensterbreite des Hervorhebungsverarbeitungsbildes derart zu verengen, dass das Ziel hervorgehoben wird.

**14.** Medizinisches Bildverarbeitungsverfahren, umfassend:
Erfassen einer Vielzahl von Zeitreihen-Röntgenbildern eines Objekts, innerhalb dessen ein sich bewegendes Ziel bereitgestellt ist, wobei das Verfahren **gekennzeichnet ist durch**:

Generieren eines Bewegungsunterdrückungsbildes auf der Grundlage von zwei oder mehr der Vielzahl von Zeitreihen-Röntgenbilder, in dem bewegungsbezogene Komponenten in Bezug auf das sich bewegende Ziel stärker unterdrückt werden als Komponenten in den zwei oder mehr der Vielzahl von Zeitreihen-Röntgenbildern, wobei in dem Bewegungsunterdrückungsbild jedes Pixel einen repräsentativen Wert der Pixelwerte der zwei oder mehr Röntgenbilder aufweist, sodass das sich bewegende Ziel unterdrückt wird;

Generieren eines Differenzbildes **durch** Differenzverarbeitung zwischen dem Bewegungsunterdrückungsbild und einem der Zeitreihen-Röntgenbilder; und

Generieren eines Hervorhebungsverarbeitungsbildes, in dem das sich bewegende Ziel in dem einem der Zeitreihen-Röntgenbilder auf der Grundlage des generierten Differenzbildes hervorgehoben wird, wobei eine Größe des Hervorhebungsverarbeitungsbildes die gleiche ist wie die des Bewegungsunterdrückungsbildes.

**15.** Medizinisches Bildverarbeitungsverfahren nach Anspruch 14, wobei:

das Erfassen der Röntgenbilder durch einen beliebigen von einem Client und einem Server ausgeführt wird, von denen beide von einem medizinischen Bildverarbeitungssystem eingeschlossen sind;

das Extrahieren der bewegungsbezogenen Komponente durch einen beliebigen von dem Client und dem Server ausgeführt wird; und

das Generieren des Hervorhebungsverarbeitungsbildes durch einen beliebigen von dem Client und dem Server ausgeführt wird.

**Revendications**

**1.** Appareil de diagnostic par rayons X comprenant :

une unité d'acquisition (21) configurée pour acquérir une pluralité d'images radiographiques en série temporelle ($I_n$) d'un objet dans lequel une cible mobile est fournie, l'appareil étant **caractérisé par** :

une unité de génération d'images de suppression de mouvement (22) configurée pour générer, sur la base de deux ou plus de la pluralité d'images radiographiques en série temporelle ($I_n$), une image de suppression de mouvement A dans laquelle des composantes liées au mouvement concernant la cible mobile sont supprimées plus que des composantes dans les deux ou plus de la pluralité d'images radiographiques en série temporelle ($I_n$), dans lequel, dans l'image de suppression de mouvement (A), chaque pixel présente une valeur représentative de valeurs de pixel des deux images radiographiques ($I_n$) ou plus de sorte que la cible mobile soit supprimée ;

**caractérisé en ce que** l'appareil comprend en outre

une unité de génération d'images par soustraction (23) configurée pour générer une image de différence ($M_n$) par traitement de différence entre l'image de suppression de mouvement (A) et l'une des images radiographiques en série temporelle ($I_n$) ; et

une unité de génération d'images de traitement de mise en évidence (24) configurée pour générer une image de traitement de mise en évidence ($SI_n$) dans laquelle la cible mobile est mise en évidence dans l'une des images radiographiques en série temporelle ($I_n$) sur la base de l'image de différence générée ($M_n$), une taille de l'image de traitement de mise en évidence ($SI_n$) étant la même que celle de l'image de suppression de mouvement (A).

**2.** Appareil de diagnostic par rayons X selon la revendication 1, dans lequel l'unité de génération d'images de traitement de mise en évidence (24) est configurée pour générer une image de traitement de mise en évidence à partir d'une image radiographique par composition de l'image radiographique et de l'image de différence.

**3.** Appareil de diagnostic par rayons X selon la revendication 1 ou 2, comprenant en outre une unité d'extraction (23) configurée pour extraire des composantes liées au mouvement dans chacune de la pluralité d'images radiographiques en soustrayant l'image de suppression de mouvement de chacune de la pluralité d'images radiographiques.

**4.** Appareil de diagnostic par rayons X selon une quelconque revendication précédente, l'unité de génération d'images de suppression de mouvement (22) étant configurée pour générer, comme image de suppression de mouvement, une image de valeur moyenne ou une image de valeur médiane de deux ou plus de la pluralité d'images radiographiques.

**5.** Appareil de diagnostic par rayons X selon une quelconque revendication précédente, dans lequel lorsqu'une irradiation aux rayons X est commutée d'activée à désactivée puis ramenée à activée, l'unité de génération d'images de suppression de mouvement (22) est configurée pour mettre à jour l'image de suppression de mouvement sur la base de l'image de suppression de mouvement générée avant d'être commutée à désactivée et de l'image aux rayons X obtenue après avoir été ramenée à activée.

6. Appareil de diagnostic par rayons X selon une quelconque revendication précédente, dans lequel lorsqu'un champ d'irradiation dans l'imagerie par rayons X de l'objet est modifié, l'unité de génération d'images de suppression de mouvement (22) est configurée pour générer l'image de suppression de mouvement du champ d'irradiation modifié de sorte que l'image de suppression de mouvement basée sur deux ou plus de la pluralité d'images par rayons X de séries temporelles imagées dans le champ d'irradiation avant le changement soit utilisée pour la partie du champ d'irradiation modifié qui chevauche le champ d'irradiation avant le changement.

7. Appareil de diagnostic par rayons X selon la revendication 2, dans lequel l'unité de génération d'images de traitement de mise en évidence (24) est adaptée pour multiplier la composante liée au mouvement extraite par un facteur afin de générer une image de mise en évidence intermédiaire, et de composer l'image de mise en évidence intermédiaire et l'image radiographique pour générer l'image de traitement de mise en évidence.

8. Appareil de diagnostic par rayons X selon la revendication 7, comprenant en outre :

   une unité d'extraction (23) configurée pour extraire des composantes liées au mouvement dans chacune de la pluralité d'images radiographiques en soustrayant l'image de suppression du mouvement de chacune de la pluralité d'images radiographiques ; et
   une unité de traitement de mise en évidence configurée pour :

   convertir l'image radiographique en une pluralité de données de bandes de fréquences ;
   attribuer le coefficient de mise en évidence à chacune de la pluralité de données de bandes de fréquences conformément à un rapport de la composante liée au mouvement extraite en coopérant avec l'unité d'extraction (23) et l'unité de génération d'images de traitement de mise en évidence (24) ; et
   générer des données d'image de l'image de mise en évidence intermédiaire pour chacune de la pluralité de bandes de fréquence sur la base du coefficient de mise en évidence attribué,
   dans lequel l'unité de génération d'images de traitement de mise en évidence (24) est configurée pour générer l'image de traitement de mise en évidence sur la base des données d'image de l'image de mise en évidence intermédiaire pour chacune de la pluralité de bandes de fréquences.

9. Appareil de diagnostic par rayons X selon l'une quelconque des revendications 1 à 8, dans lequel l'unité de génération d'image de traitement de mise en évidence (ESIn) (24) est adaptée pour réduire une largeur de fenêtre de l'image de traitement de mise en évidence ($SI_n$) de sorte que la cible soit mise en évidence.

10. Appareil de diagnostic par rayons X selon l'une quelconque des revendications 1 à 9, dans lequel les composants liés au mouvement incluent un mouvement dérivé d'au moins une parmi une pulsation et/ou une respiration de l'objet et/ou, lorsque la cible se déplace à l'intérieur de l'objet, un mouvement dérivé du mouvement de la cible.

11. Appareil de diagnostic par rayons X selon l'une quelconque des revendications 1 à 10, dans lequel :
l'unité de génération d'images de suppression de mouvement (22) est configurée pour générer une image de valeur représentative comme image de suppression de mouvement dans laquelle chaque pixel a une valeur représentative de valeurs de pixel dans les deux images radiographiques ou plus, sur la base des deux images radiographiques ou plus de la pluralité d'images radiographiques de séries temporelles et l'unité de génération d'images de traitement de mise en évidence (24) est configurée pour générer l'image de traitement de mise en évidence de la cible représentée dans l'image de différence en ajoutant l'image de différence générée et l'image radiographique correspondant à l'image de différence.

12. Appareil de diagnostic par rayons X selon la revendication 11, dans lequel l'unité de génération d'images de traitement de mise en évidence (24) est configurée pour générer l'image de traitement de mise en évidence en ajoutant l'image de différence multipliée par un facteur et l'image radiographique correspondant à l'image de différence.

13. Appareil de diagnostic par rayons X selon l'une quelconque des revendications 11 à 12, dans lequel l'unité de génération d'images de traitement de mise en évidence (24) est configurée pour réduire une largeur de fenêtre de l'image de traitement de mise en évidence de sorte que la cible soit mise en évidence.

14. Procédé de traitement d'images médicales, comprenant :

l'acquisition d'une pluralité d'images radiographiques en série temporelle d'un objet dans lequel une cible mobile est fournie, le procédé étant **caractérisé par** :

la génération, sur la base de deux ou plus de la pluralité d'images radiographiques en série temporelle, d'une image de suppression de mouvement dans laquelle des composantes liées au mouvement concernant la cible mobile sont supprimées plus que des composantes dans les deux ou plus de la pluralité d'images radiographiques en série temporelle, dans lequel, dans l'image de suppression de mouvement (A), chaque pixel présente une valeur représentative de valeurs de pixel des deux images radiographiques ou plus de sorte que la cible mobile soit supprimée ;

la génération d'une image de différence par traitement de différence entre l'image de suppression du mouvement et l'une des images radiographiques en série temporelle ; et

la génération d'une image de traitement de mise en évidence dans laquelle la cible mobile est mise en évidence dans l'une des images radiographiques en série temporelle sur la base de l'image de différence générée, une taille de l'image de traitement de mise en évidence étant la même que celle de l'image de suppression du mouvement.

15. Procédé de traitement d'images médicales selon la revendication 14, dans lequel

l'acquisition des images radiographiques est exécutée par l'un quelconque d'un client et d'un serveur, tous deux inclus dans un système de traitement d'images médicales ;

l'extraction des composantes liées au mouvement est exécutée par l'un quelconque du client et du serveur ; et

la génération de l'image de traitement de mise en évidence est exécutée par l'un quelconque du client et du serveur.

1

MEDICAL IMAGE PROCESSING APPARATUS — 10

11 INPUT INTERFACE

12 DISPLAY

13 MEMORY

14 NETWORK CONNECTING CIRCUIT

15 PROCESSING CIRCUITRY

21 ACQUISITION FUNCTION

22 MOTION SUPPRESSION IMAGE GENERATION FUNCTION

23 EXTRACTION FUNCTION

24 EMPHASIS PROCESSING IMAGE GENERATION FUNCTION

100

101 X-RAY DIAGNOSTIC APPARATUS

102 IMAGE SERVER

FIG. 1

# FIG. 2

FIG. 3

IMAGE BEFORE
PROCESSING $I_n$

31

32

33

EMPHASIS
PROCESSING
IMAGE $ESI_n$

31

32

33

# FIG. 4

PROCESSING

WIDER IRRADIATION FIELD

NEWLY START PROCESSING

CONTINUE
PROCESSING
(RESIZE)

CHANGE POSITION (PANNING)

NEWLY START PROCESSING

CONTINUE PROCESSING
(COODINATE TRANSFORMATION)

CONTINUE
PROCESSING
(COODINATE
TRANSFORMATION)

FIG. 5

IMAGE BEFORE PROCESSING $I_n$

EMPHASIS PROCESSING IMAGE $ESI_n$

41

# FIG. 6

START

ACQUIRE MULTIPLE TIME-SERIES X-RAY IMAGES — S1

GENERATE MOTION SUPPRESSION IMAGE
BASED ON MULTIPLE X-RAY IMAGES — S2

EXTRACT TARGET FROM EACH X-RAY IMAGE
BASED ON MOTION SUPPRESSION IMAGE — S3

MULTIPLY EXTRACTED OBJECT
BY EMPHASIS COEFFICIENT — S4

COMPOSITE TARGET WITH X-RAY IMAGE
OF CORRESPONDING FRAME — S5

IMAGE PROCESSING OF THE COMPOSITE IMAGE
SO THAT TARGET IS EMPHASIZED — S6

END

FIG. 7

FIG. 8

1

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015139394 A1

- US 2018082420 A1